Europäisches Patentamt

(19) European Patent Office

Office européen des brevets

(11) Publication number : **0 447 259 A1**

# (12) EUROPEAN PATENT APPLICATION

(21) Application number : **91302258.8**

(22) Date of filing : **15.03.91**

(51) Int. Cl.⁵ : **C07C 25/13, C07C 17/22, C07C 245/20, C07C 211/52**

(30) Priority : **16.03.90 GB 9006013**

(43) Date of publication of application :
**18.09.91 Bulletin 91/38**

(84) Designated Contracting States :
**AT BE CH DE DK ES FR GB GR IT LI LU NL SE**

(71) Applicant : **ISC CHEMICALS LIMITED**
**33 Ashley Place**
**London SW1P 1LS (GB)**

(72) Inventor : **Wotton, David Edward Mudge**
**66 Roman Way**
**Stoke Bishop, Bristol BS9 1SS (GB)**
Inventor : **Russell, James John**
**5 Wellington Walk**
**Westbury on Trym, Bristol BS10 5ET (GB)**

(74) Representative : **Collier, Jeremy Austin Grey et al**
**J.A.Kemp & Co. 14, South Square Gray's Inn**
**London WC1R 5EU (GB)**

(54) Process for preparing chloro-difluorobenzene.

㊐   1-Chloro-3,4-difluoro-benzene, an intermediate in the production of certain quinolone drugs, is produced by converting a chlorofluoro-aniline into the corresponding diazonium fluoborate, and pyrolysing the latter to give the 1-chloro-3,4-difluorobenzene.
5-Chloro-2-fluorophenyldiazonium fluoborate is a new compound.

EP 0 447 259 A1

# PROCESS FOR PREPARING CHLORO-DIFLUOROBENZENE

This invention relates to the synthesis of chlorodifluorobenzenes useful as intermediates for quinoline drugs, which have anti-bacterial properties.

British Patent No. 830,832 (ICI Ltd.) published 22nd March 1960, describes and claims substituted quinoline derivatives of generally N-alkyl quinolone structure with the heterocyclic ring substituted by alkyl and carboxyl groups.

More recently there has been a requirement for quinolone based drugs wherein the carbocyclic ring in the quinoline nucleus is substituted by halogen atoms, especially fluorine and chlorine atoms. Such compounds are disclosed, inter alia, in European Patent specifications 000,203 (Ciba-Geigy) and 0,303,291 A2 (Asahi Glass), Belgian Patent 863,429 (Kyorin), French Patents 2,210,413 and 2,257,292 (Dainippon Pharmaceuticals), and US Patent 4,730,000 (Abbott Laboratories).

A valuable precursor for making some halogen substituted N-alkyl quinolone derivatives e.g. as referred to in US Patent 4,730,000 is 1-chloro-3,4-difluorobenzene. This can easily be converted into the key intermediate 2-chloro-4,5-difluorobenzoic acid (by acetylation and subsequent oxidation of the intermediate acetophenone) and this benzoic acid can then be converted into a substituted quinolone - e.g. by the methods described in European Patent Application 0,303,291 A2.

However, 1-chloro-3,4-difluorobenzene has proved to be extremely difficult to synthesise on a commercial scale. Methods published in the literature are as follows:

## Method (a)

- Diazotisation of 3,4-difluoroaniline in aqueous hydrochloric acid, with aqueous sodium nitrite, followed by reaction with cuprous chloride/aqueous HCl. (G C Finger, M J Gortatowski, R H Shiley and R H White, J.Amer.Chem.Soc., 1959, 81, 94-101).

## Method (b)

- Reaction of 1,2,4-trichlorobenzene with potassium fluoride in dimethylsulphone. (R H Shiley, D R Dickerson and G C Finger, J.Fluorine.Chem., 1972-73, 2 (1) 19-26).

## Method (c)

- Reaction of 1,2,4-trichlorobenzene with a nitrosyl fluoride/hydrogen fluoride complex mixture over cobalt fluoride at 420° (±15°). (J Gordon, H R Nychka and C Woolf, French Patent No 1,385,681, to Allied Chemical Corporation).

## Method (d)

- Reaction of chloroaromatic compounds with silver difluoride. (A Zweig, R G Fischer and J E Lancaster, J.Org.Chem., 1980, 45 (18), 3597-603).

## Method (e)

- Pyrolysis of vinyl fluorocyclobutanes. (F J Weigert, US Patent No 4,754,084).

Disadvantages of these processes are as follows:

## Method (a)

While the process of conversion of 3,4-difluoroaniline into 1-chloro-3,4-difluoroaniline by the Sandmeyer reaction can be carried out on a large scale without significant difficulty, the starting material, 3,4-difluoroaniline, cannot be prepared economically on the tonnage scale by known commercial processes, and is, therefore, expensive and difficult to acquire.

## Method (b)

1,2,4-Trichlorobenzene is an easily available starting material, but the yield of 8% is too low to be commercially viable, the product is mixed with isomers which make it difficult to isolate, and the process has to be carried out in expensive pressure equipment, which, since corrosion from chloride ion would be expected, has to be made from expensive materials.

## Method (c)

The reagents are toxic and expensive, and yields of the desired product are poor. The process is, therefore, not commercially viable.

## Method (d)

Silver difluoride can only be made using elemental fluorine and is expensive and difficult to handle. The process is complex and consists of several stages, making it non-competitive.

## Method (e)

In this process, the raw materials are sophisticated and expensive, and the selectivity for the desired product is low, again giving a process which is not competitive.

There is, therefore, a requirement for an economically viable route to 1-chloro-3,4-difluorobenzene for use as an intermediate for the production of specialised fluorinated quinolone anti-bacterial drugs, for which there is an increasing demand.

The present invention provides a process for the preparation of 1-chloro-3,4-difluorobenzene which comprises converting a chlorofluoro aniline having a fluorine atom in the 2-position with respect to the amino group, and a chlorine atom in the 4- or 5- position with respect to the amino group into the corresponding diazonium fluoborate, and pyrolysing this product to produce the required chlorodifluorobenzene.

The route from the known compound 5-chloro-2-fluoroaniline is attractive because this aniline can easily be prepared on a tonnage scale from widely available starting materials in a two stage process:

(a) reaction of 2,5-dichloronitrobenzene with potassium fluoride, preferably using one equivalent proportion in an aprotic solvent, to give 5-chloro-2-fluoro nitrobenzene in good yield; and

(b) reduction of the resulting 5-chloro-2-fluoronitrobenzene to 5-chloro-2-fluoroaniline in known manner, preferably using existing commercial processes, e.g. the Béchamp iron reduction process, or catalytic hydrogenation. These processes go in near quantitative yield.

Alternatively, but less preferably, 4-chloro-2-fluoroaniline can be prepared by a three stage process on a tonnage scale from easily available starting materials:

(a) reaction of 2,4-dichloronitrobenzene with one equivalent proportion of potassium fluoride in an aprotic solvent to give a mixture of 2-chloro-4-fluoronitrobenzene and 4-chloro-2-fluoronitrobenzene in good yield;

(b) separation of the two isomeric chlorofluoronitrobenzenes by fractional crystallisation; and

(c) reduction of the resulting 4-chloro-2-fluoronitrobenzene to 4-chloro-2-fluoroaniline using existing commercial processes, eg. the Béchamp iron reduction process, or catalytic hydrogenation, in near quantitative yield.

According to the process of this invention, either 4-chloro-2-fluoroaniline or 5-chloro-2-fluoroaniline is converted into the corresponding diazonium fluoborate by dissolving the chlorofluoroaniline in aqueous mineral acid (eg. hydrochloric acid, sulphuric acid, or fluoboric acid) at a temperature in the range -20° to 40°C, and adding a source of nitrite ions (eg aqueous sodium nitrite or potassium nitrite, nitrosyl sulphate, nitrosyl chloride, or an organic nitrite) to give the 4- or 5-chloro-2-fluorophenyl diazonium salt of the relevant acid. To this solution or suspension is added a source of fluoborate ion (eg. fluoboric acid, or sodium, potassium, or ammonium fluoborate) and the 4- or 5-chloro-2-fluorophenyl diazonium fluoborate salt, which is relatively insoluble, precipitates. This is filtered off, washed, and dried at ambient temperature in vacuo.

The resulting dried, powdered 4- or 5-chloro-2-fluorophenyl diazonium fluoborate is pyrolysed either alone, or in the presence of a liquid medium, preferably a hydrocarbon or halogenated hydrocarbon (eg. a polyhalogenated benzene, xylene, or mesitylene), at atmospheric pressure (or at subatmospheric pressure, or superatmospheric pressure, according to the most convenient procedure). The desired product, 1-chloro-3,4-difluorobenzene distils out, and can be condensed and purified (eg. by fractional distillation).

5-Chloro-2-fluorophenyl diazonium fluoborate is a new compound and as such within the scope of the present invention. Operation of the new process using this intermediate is especially advantageous since it has been found to be significantly less likely to cause irritation and sensitization of the skin than its isomer 4-chloro-2-fluorophenyl diazonium fluoborate.

The following Examples illustrate the invention.

Example 1 (from 5-chloro-2-fluoroaniline)

(a) Precipitation of the diazonium fluoborate salt

5-Chloro-2-fluoroaniline (100g) was added slowly with stirring to a mixture of concentrated hydrochloric acid (184 ml) and water (64.4ml). A mildly exothermic reaction ensued, and the mixture was stirred at ca. 50°C for half-an-hour, and then cooled to -5°C. A solution of sodium nitrite (62.6g) in water (46ml), was then slowly added to the mixture at -5°C to 0°C. Fluoboric acid (40%, 207 ml) was added to the clear solution obtained and a pale yellow slurry resulted. This was filtered, and the precipitated 5-chloro-2-fluorophenyl diazonium fluoborate was washed with a mixture of equal volumes of fluoboric acid and water, followed by methanol and ether. The salt was then dried in vacuo at ambient temperature, to give 145g of pure 5-chloro-2-fluorophenyl diazonium fluoborate, (87% yield), mp 210°C (with decomposition).

(b) Thermal decomposition

120g of the dry, 5-chloro-2-fluorophenyl diazonium fluoborate was cautiously heated in a 1 1. round bottomed flask over a gentle gas flame. The distillate was condensed and analysed. 44g of a colourless oil was collected. Gas chromatographic analysis coupled to mass spectrometry showed this product to have the following composition:

| Compound | Concentration (%) |
|---|---|
| 1,2,4-trifluorobenzene | 0.6 |
| 1-chloro-3,4-difluorobenzene | 96.7 |
| isomer of above | 0.2 |
| 2,5-dichlorofluorobenzene | 1.8 |
| dichlorotrifluorobiphenyl | 0.2 |
| isomer of above | 0.1 |
| isomer of above | 0.2 |

Thus, from 120g of 5-chloro-2-fluorophenyldiazonium fluoborate (0.49 gmoles) were obtained 42.5g (0.29 gmoles) of 1-chloro-3,4-difluorobenzene, a yield of 58%.

Example 2 (from 4-chloro-2-fluoroaniline with dry decomposition of the fluoborate salt)

(a) Precipitation of the diazonium fluoborate salt

4-Chloro-2-fluoroaniline (160g) was added slowly with stirring to a mixture of concentrated hydrochloric acid (294ml) and water (103ml). A mildly exothermic reaction ensued, and the mixture was stirred at ca. 50°C for half-an-hour, and then cooled to -5°C. A solution of sodium nitrite (100g) in water (74ml) was then slowly added to the mixture at -5°C to 0°C. Fluoboric acid (40%, 331ml) was added to the clear solution and a pale yellow slurry resulted. This was filtered, and the precipitated 4-chloro-2-fluorophenyl diazonium fluoborate was washed with a mixture of equal volumes of fluoboric acid and water, followed by methanol and ether. The salt was then dried in vacuo at ambient temperature, to give 166g of pure 4-chloro-2-fluorophenyl diazonium fluoborate, (61.8% yield), mp 170-175°C.

(b) Thermal decomposition

150g of the dry 4-chloro-2-fluorophenyl diazonium fluoborate was cautiously heated in a 500ml round bottomed flask in an electric mantle, to ca 200°C. An exothermic reaction developed, and the resulting distillate was condensed and analysed. 44g of a colourless oil were collected. Gas chromatographic analysis coupled to mass spectrometry showed this product to have the following composition:

4

| Compound | Concentration |
|---|---|
| | (%) |
| 1,2,4-trifluorobenzene | 0.5 |
| 1-chloro-3,4-difluorobenzene | 94.1 |
| 2,5-dichlorofluorobenzene (probable) | 3.1 |
| 2,4-dichlorofluorobenzene (probable) | 0.2 |
| Other smaller peaks (<0.5%) | 2.1 |

Thus, from 150g of 4-chloro-2-fluorophenyldiazonium fluoborate (0.61g moles) were obtained 41.4g (0.28g moles) of 1-chloro-3,4-difluorobenzene, a yield of 46%.

Example 3: (from 4-chloro-2-fluoroaniline with decomposition of the fluoborate salt in a 1,2,4-trichlorobenzene slurry).

4-Chloro-2-fluorophenyldiazonium fluoborate (dry powder prepared by the method described in Example 2, 84.3g) was slurried at ambient temperature in 1,2,4-trichlorobenzene (546g) and the slurry was slowly fed in small portions into 1,2,4-trichlorobenzene (141g) maintained at 210°C. The vapours evolved were cooled to about 15°C, and the condensate was fractionally distilled to give 1-chloro-3,4-difluorobenzene, 13.4g., a yield of 26%.

**Claims**

1. A process for the preparation of 1-chloro-3,4-difluorobenzene which comprises converting a chlorofluoro aniline having a fluorine atom in the 2- position with respect to the amino group, and a chlorine atom in the 4- or 5- position with respect to the amino group into the corresponding diazonium fluoborate, and pyrolysing this product to produce the required chloro-difluorobenzene.

2. A process as claimed in claim 1 wherein the said aniline is dissolved in aqueous mineral acid with addition of nitrite ions to produce a fluorophenyl diazonium salt of the relevant acid, and a source of fluoborate ions is then added to produce the diazonium fluoborate.

3. A process as claimed in claim 1 or 2 wherein the diazonium salt is formed at -20° to +40°C.

4. A process as claimed in any one of claims 1 to 3 wherein the pyrolysis is carried out in the absence of a solvent.

5. A process as claimed in any one of claims 1 to 3 wherein the pyrolysis is carried out in a hydrocarbon or halogenated hydrocarbon medium.

6. A process as claimed in any one of claims 1 to 5 wherein the starting material is 5-chloro-2-fluoro-aniline.

7. A process as claimed in claim 6 wherein the 5-chloro-2-fluoro-aniline is obtained from 2,5-dichloronitrobenzene by reaction with potassium fluoride to give 5-chloro-2-fluoro-nitrobenzene followed by reduction of the latter to give the required aniline.

8. 5-Chloro-2-fluorophenyl diazonium fluoborate.

European Patent
Office

## EUROPEAN SEARCH REPORT

Application Number

EP 91 30 2258

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 355 719 (ASAHI GLASS CO.)<br>* Comparative example 1 *<br>----- | | C 07 C 25/13<br>C 07 C 17/22<br>C 07 C 245/20<br>C 07 C 211/52 |
| | | | TECHNICAL FIELDS SEARCHED (Int. Cl.5) |
| | | | C 07 C 17/00 |
| | The present search report has been drawn up for all claims | | |

| Place of search | Date of completion of the search | Examiner |
|---|---|---|
| THE HAGUE | 21-05-1991 | VAN GEYT J.J.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
-------------------------------------------
& : member of the same patent family, corresponding document

EPO FORM 1503 03.82 (P0401)

6